(19) Europäisches Patentamt

European Patent Office

Office européen des brevets

(11) EP 4 696 368 A1

(12) EUROPEAN PATENT APPLICATION

(43) Date of publication:
18.02.2026 Bulletin 2026/08

(21) Application number: 24194441.2

(22) Date of filing: 14.08.2024

(51) International Patent Classification (IPC):
A61N 1/37 *(2006.01)* A61N 1/378 *(2006.01)*

(52) Cooperative Patent Classification (CPC):
A61N 1/3787; A61N 1/3718

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: BIOTRONIK SE & Co. KG
12359 Berlin (DE)

(72) Inventors:
• VON ARX, Jeffrey A.
Lake Oswego, 97035 (US)
• WHITTINGTON, R. Hollis
Portland, 97202 (US)
• MUESSIG, Dirk
West Linn, 97068 (US)
• FRYER, Alan
Portland, 97202 (US)

(74) Representative: Biotronik Corporate Services SE
Corporate Intellectual Property
Sieversufer 9
12359 Berlin (DE)

(54) **WIRELESS CHARGER FOR AN IMPLANTABLE MEDICAL DEVICE**

(57) A wireless charger (20) and implantable medical device, IMD (1) that are paired to allow the charger to wirelessly charge a rechargeable battery (17) of the IMD (1) when the latter is subcutaneously implanted in a patient in tissue (72) under the skin (70).

For wireless charging, power is transferred inductively from transmit coils (23) in the charger (20) to receive coils (13) in the IMD (1). To align the transmit and receive coils (23, 13), the charger (20) and IMD (1) are provided with respective magnets (21, 7) arranged centrally within their associated coils (23, 13). Magnetic attraction between the magnets when the charger is positioned over the IMD (1) site provides tactile feedback when alignment is achieved. Moreover, once the coils are aligned, the continued magnetic attraction between the magnets holds the alignment position during subsequent charging.

**FIG. 2A**

## Description

The invention relates to a charger for wirelessly charging an implantable medical device (IMD) implanted in a patient.

An IMD with on-board electronics will typically require an electrical power source in the form of a battery. If the IMD is implanted close to the skin, i.e., subcutaneously, which is typically the case for neurostimulation devices such as a spinal cord stimulator (SCS), it is convenient to fit a rechargeable battery (i.e. secondary cell) that is charged *in situ* with a suitable wireless charger that is placed on the skin by the patient or a caregiver over the IMD to provide for contactless charging. For this purpose, the IMD is provided with pick-up coils, referred to as receive coils, to allow the rechargeable battery to be wirelessly charged through inductive coupling to a matched set of coils in the charger, referred to as transmit coils.

While the use of a rechargeable battery allows a much smaller battery to be used compared with a non-rechargeable battery (i.e. primary cell), it self-evidently must be recharged from time-to-time and the responsibility for this task lies with the patient. Charging may be needed at intervals of a week or sometimes more frequently. Each charge may take thirty minutes, assuming the transmit and receive coils are well aligned. Charging times are substantially longer if the coils are misaligned.

For charging, the wireless charger must be positioned over the IMD site so that the transmit coils of the charger are aligned with the receive coils of the IMD. Alignment by feeling the bump of the IMD on the skin is not reliable, since the IMD receive coils are often accommodated off center in the IMD, e.g., in the header or at one end of the can. To aid this alignment process, it is known to use a flashing indicator light and/or an audible beeping sound to indicate when the charger is optimally placed.

The rate at which energy can be transmitted to the IMD during charging is limited by the need to avoid overheating the IMD and also the tissue between the charger and IMD bearing in mind that prolonged exposure to temperatures above 43°C causes tissue damage. The charging process therefore needs to be controlled to take account of temperature readings taken at the IMD to ensure a certain temperature or thermal dose is not exceeded, for example to comply with ISO 14708-3:2017.

The rate of energy transfer across an inductive link provided by the wireless charger is defined by a coupling coefficient, which depends on several parameters including the distance between the transmit and receive coils, the alignment between the transmit and receive coils, the size mismatch between the transmit and receive coils and the quality factor of the coils.

It is usual to oversize the transmit coils relative to the receive coils so that there is inductive power transfer to the IMD over quite a large range of misalignment between the axes of the coils. In effect, the design assumes that perfect or even very close alignment between the coils is not achievable. The oversizing of the transmit coils therefore ensures that IMD charging can be performed reliably by the patient. Specifically, in current commercial systems, the charger's transmit coils typically have a diameter of two to three times that of the IMD's receive coils. However, the oversizing of the transmit coils has the drawback of significantly decreasing coupling efficiency. More specifically, the coupling coefficient decreases with the square of the mismatch in coil diameters. For example, if the charger's transmit coils are twice the diameter of the IMD's receive coils, the coupling coefficient is four times lower compared to what it would be if the transmit and receive coils were the same size.

According to first aspect of the disclosure there is provided an implantable medical device (IMD), comprising:

an electronics unit for controlling the IMD,
a rechargeable battery connected to power the electronics unit,
receive coils connectable by the electronics unit to charge the rechargeable battery and arranged to receive an electromagnetic field to provide electrical energy for wirelessly charging the rechargeable battery, and
a magnet configured to provide a magnetic field to couple with a corresponding magnet of an external charger when placed adjacent the IMD.

Preferably, according to an embodiment, the magnet is an electromagnet.

Certain embodiments of the invention may provide one or more of the following advantages.

Charging time is reduced, since the coupling coefficient is increased by the transmit coils and receive coils being similarly dimensioned, which is enabled by the fact that good alignment is guaranteed by the magnet locking.

Charging time is reduced, since the good alignment between the transmit coils and receive coils reduces IMD heating effects, specifically eddy current heating of the metal casing of the IMD can. The reduced heating means it is less likely that transmit coil power will need to be reduced during charging to keep IMD temperatures below acceptable limits, this being done typically by duty cycling the current applied to the transmit coils.

To initiate a charging process, the charger automatically aligns to the IMD, by which is meant that the centers of the transmit and receive coils align with each other, through the magnetic attraction between their respective magnets. The two magnets 'finding' each other provides intuitive tactile feedback to the patient or caregiver who is attempting to align the charger correctly over the implanted IMD. This is intuitive and has certain advantages over known feedback via an indicator light or sound signal.

The charger remains aligned with the IMD throughout the charging process through the charger being held in place by the magnetic field between the respective magnets of the charger and IMD. With a sufficiently strong magnetic field, the patient is free to move around without the charger falling off under gravity.

An improved charging experience for the patient makes it more likely that the patient will stay compliant by charging the IMD when needed.

In certain embodiments the IMD magnet is an electromagnet. Using an electromagnetic is advantageous for magnetic resonance imaging (MRI) compliance, since the electromagnet can be energized selectively only for charging and thus does not present a hazard to the patient when exposed to high magnetic fields when in an MRI scanner.

This invention will now be further described, by way of example only, with reference to the accompanying drawings.

Fig. 1　　is a schematic diagram of an IMD arranged in a medical device communication system (MDCS).

Fig. 2A　　is a schematic cross-sectional drawing of a in IMD and charger embodying the invention with the IMD implanted in a patient.

Fig. 2B　　is a schematic front view of the charger of Fig. 2A.

Fig. 2C　　is a schematic front view of the IMD of Fig. 2A.

In the following detailed description, for purposes of explanation and not limitation, specific details are set forth in order to provide a better understanding of the present disclosure. It will be apparent to one skilled in the art that the present disclosure may be practiced in other embodiments that depart from these specific details.

In particular, the embodiments described in the following are described by way of example only in relation to an SCS system.

An SCS device is a type of IMD that is used for chronic pain therapy of a patient. An SCS device uses electrical current sent through electrodes on one or more leads implanted in the epidural space dorsal to the spinal cord in an SCS device. Control parameters include at least: pulse width, frequency, amplitude, and electrode selection. In a conventionally constructed IMD for SCS there are two principal units: a header and a can. The header is the interface area for conductive leads for SCS which are embedded in a nerve trunk of the patient. These carry therapeutic stimulation pulses and also may be used for sensing evoked compound action potential (ECAP) signals. The header casing is typically of a plastics material or an epoxy. The can contains the electronics, sensors, and communications hardware, as required for external communication using a suitable wireless or near-field protocol. The can also contains the rechargeable battery. The can has a metal outer casing, made of a biocompatible metal, typically titanium or a titanium alloy.

An IMD typically forms part of a medical device communication system (MDCS) that is configured to upload clinical data from the IMD on a continual basis via a MDCS to a remotely located data repository, which may be a data center. In an SCS device, clinical data includes both patient data relating to physiological monitoring of a patient's health, for example as collected by sensors of the IMD, and device data related to status and operation of the IMD, for example data logging each time an SCS device is used and at what stimulation level in terms of electrical pulse width and pulse frequency as well as control signals issued to the SCS device to optimize delivery of pain relief. These clinical data can then be accessed by health care staff accessing the data center using suitably designed software applications. Treatment plans and, if necessary, interventions can then be decided upon based on analysis of these clinical data. The analysis may be expert analysis by a health care professional or computer-automated analysis with a software program, e.g., running on computing resource at a neuro-service data center, or a combination of both.

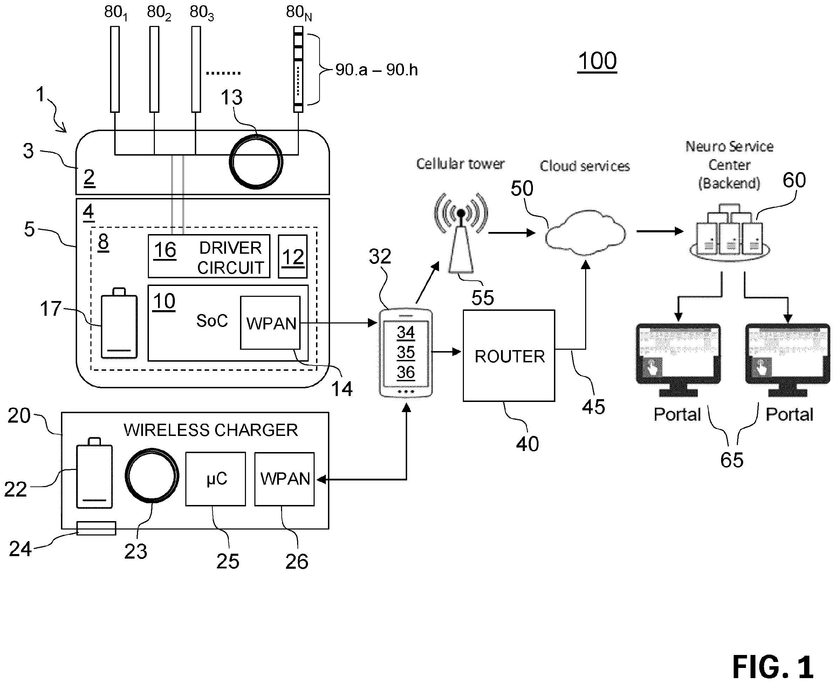

Figure 1 shows a standard architecture of a MDCS 100 for communication between an IMD 1 implanted in a patient and a remotely located data center 60, which is accessible to health care staff using suitable application software. The patient is provided with a patient remote controller 32 (hereinafter patient remote) loaded with a suitable application (so-called app) providing the patient with a user interface to interact with the IMD 1.

The IMD 1 comprises a header 2 and a can 4 which have respective outer casings 3 and 5. The header casing 3 is non-metal, e.g. a hard plastics material, epoxy or ceramic. The can casing is made of a biocompatible metal, typically titanium or a titanium alloy. The can 4 includes therapeutic components for patient treatment, such as an SCS driver circuit 16 (or another IMD stimulator) and optionally an SCS sensing circuit for sensing ECAP signals (not shown). The can 4 also contains an electronics unit 8 including a system-on-a-chip (SoC) 10 with control electronic components for controlling the IMD 1, e.g., a microcontroller. One task of the SoC 10 is to deliver control signals to the electrode driver circuit 16 according to a treatment plan. The SoC 10 also includes a wireless personal area network (WPAN) transceiver 14 for external wireless communication, e.g., to the patient remote controller 32 as illustrated. A rechargeable battery 17 is provided for powering the electronics unit 8 and for providing power to the driver circuit 16. The rechargeable battery 17 is wirelessly charged through receive coils 13 which are illustrated placed in the header 2 but in other embodiments are placed in the can 4 or in a separate non-metal housing attached to the can (not shown). The motivation for not accommodating the receive coils in the can is to reduce heating of the metal casing of the can that is caused by eddy currents generated by the time-varying magnetic field generated by the transmit coils of the charger. A significant amount of eddy current heating of the metal casing of the can does however still occur even when the receive coils are moved out of the can.

One or more temperature sensors 12 are provided for measuring temperature at locations in the IMD 1 that are expected to become hottest during wireless charging. These locations may include the proximal side of the metal casing 5 (i.e. the side of the casing which is closest to the charger coil during charging, the receive coils 13 and the rechargeable battery 17.

A wireless charger 20 is provided for charging the rechargeable battery 17 of the IMD 1 when the IMD 1 is implanted in a patient. The charger 20 allows charging to take place in a contactless manner by resonant induction. For charging, the charger 20 is placed on the patient's skin adjacent the implanted IMD 1. More specifically, the charger 20 is provided with transmit coils 23 which are matched to the receive coils 13 in the IMD 1.

Charger operation is controlled by a controller 25, such as a microcontroller. The charger also includes a WPAN transceiver 26 for external communication, for example with the patient remote controller 32 as illustrated. Temperature signals or values obtained by IMD temperature sensors 12 are transmitted to the charger 20, for example via transmission from the IMD 1 with WPAN transceiver 14 to the charger 20, or with WPAN transceiver 14 to the patient remote controller 32 and from the patient remote controller 32 to the charger 20. Another option (not illustrated) is to provide a direct near-field communication link between the IMD 1 and charger 20 over which temperature readings from the IMD 1 are communicated directly to the charger 20.

The charger 20 is provided with an external power connection 24, for example an external power jack, to power the charger 20 externally using a power cord, e.g., from a battery pack that can be carried on a belt. Optionally, the charger 20 may have its own rechargeable battery 22 but if this is the case, it will need to be relatively light to keep the weight of the charger 24 small so it will not be the energy source for charging but rather provide other functions when the charger 20 is not connected to an external power source such as wireless communications.

The IMD header 2 is attached to a lead arrangement comprising multiple leads $80_1$ to $80_N$ with each lead incorporating multiple electrodes 90.a to 90.h. The leads 80 attach to the header via ports (not shown) provided in the header 2. In the illustrated example, there are multiple leads but in other examples there may be only one lead. The electrode driver circuit 16 provides a suitable drive current to each (or a selectable subset) of the electrodes 90.a to 90.h on each (or a selectable subset) of the multiple leads $80_1$ to $80_N$ according to a set of control parameters that follow a treatment plan as controlled by the SoC 10.

The patient remote 32 may, for example, be a smartphone with wireless transceivers 34, 35, 36 respectively for WPAN (e.g. BLE), LPWAN, cellular (e.g., 4G/LTE/5G) and WLAN communication. If a smartphone is used, this is a smartphone that is possessed, e.g., owned, by the patient in which the IMD 1 is implanted and on which a software application ('app') is installed. The app is then a so-called Software as a Medical Device (SaMD) which is defined by the United States Food and Drug Administration (FDA) as software intended to be used for one or more medical purposes that perform these purposes without being part of a hardware medical device.

The cellular transceiver 35 and WLAN transceiver 36 provide two different data communication paths for the patient remote 32 to upload clinical data from the IMD 1 via an internet connection 50 to a remotely located data center 60 (hereinafter also called "backend 60") acting as repository for storage of clinical data and/or as a host for the services, for example a neuro-service data center. The remote's WLAN transceiver 36 can upload data to the backend 60 via a router 40 and an outer internet connection45 (e.g. telephone network, Ethernet connection, cable TY modem, satellite service) using a wired internet connection 50. The internet connection 50 may provide access to one or more distributed networks and/or cloud services. The remote's cellular transceiver 35 can upload data to the backend 60 via one or more cellular network base stations 55 (cellular towers), for example LPWAN-capable cellular network base station. In some cases, instead of a public communication network, a dedicated point-to-point transmission, e.g., via a dedicated telephone line, may be provided for uploading clinical data. In all these scenarios, uploading of clinical data from the IMD 1 to the backend 60 takes place via the intermediary of the patient remote 32, the latter thereby acting as a relay device.

Health care staff, such as health care professionals (HCPs), clinical specialists and representatives and remote care team members have access to the backend 60 via suitable portals 65 with the aid of a software application running on the backend 60 and/or the portal 65 to provide the necessary user interfacing, diagnostics and so forth. At least one portal 65 is provided by at least one workstation for health care staff to access a data center, e.g., the backend 60. Analysis and diagnostic software may also be run at the backend 60 to analyze clinical data from individual patients or groups of patients.

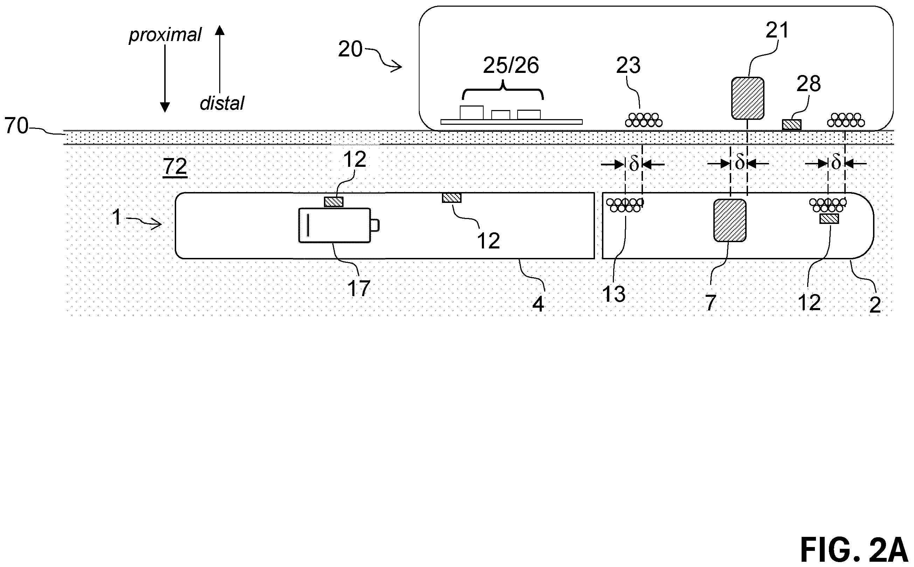

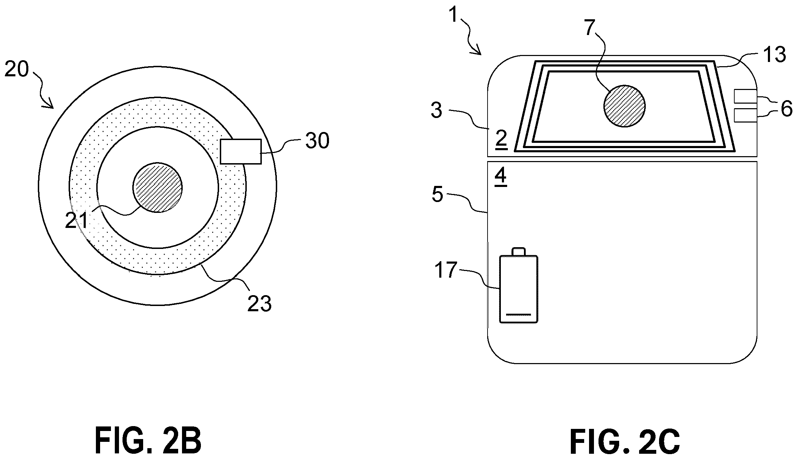

Figure 2A is a schematic cross-sectional drawing of a in IMD 1 and charger 20 embodying the invention with the IMD 1 implanted subcutaneously in a patient in a patient's subcutaneous tissue 72 under the patient's skin 70. The charger 20 is shown in position for wirelessly charging the IMD 1 but laterally offset by a distance 'δ' from perfect alignment. Figure 2B is a schematic front view of the charger 20. Figure 2C a schematic front view of the IMD 1. The IMD header 2 and can 4 are shown, as are the header's lead attachment ports 6. In Figure 2A the charger 20 is shown with its transmit coils 23 aligned with the IMD's receive coils 13. The receive coils 13 are shown as being accommodated in the header 2 but they may be in the can 4 or a separate housing as previously mentioned. During charging of an implanted IMD 1, the charger 20 is arranged with a proximal surface in contact with the patient's skin 70 over the implanted IMD 1.

The IMD 1 and charger 20 are provided with paired magnets 7, 21 respectively. The magnets 7, 21 are arranged, configured, and operated so that an attractive force is created between the magnets 7, 21 which holds the charger 20 in its correct charging position over the implanted IMD 1, i.e., so that the respective receive and transmit coils 13, 23 are aligned. In the position shown in Figure 2A, there would be a corrective attractive magnetic force component arising from the lateral displacement between the respective principal magnetic axes of the magnets 7, 21 that would act to shift the charger 20 parallel to the surface of the skin so as to reduce the misalignment distance '$\delta$' to zero, i.e., to the left in the illustrated misaligned position.

The magnets 7, 21 are thus preferably arranged centrally within their associated coils 13, 23. The act of the patient moving the charger 20 over the IMD site results provides helpful tactile feedback to the patient as the IMD magnet 7 'finds' the charger magnet 21, since then the patient can feel when the charger 20 is optimally placed for charging. In the preferred embodiment, the charger 20 is small and light enough that the mutual magnetic force between the magnets 7, 21 will snap the charger 20 into the optimal position once the charger 20 is close to alignment, and, further, that the magnetic field will hold the charger 20 in place over the implanted IMD 1. This will significantly improve the charging process by automatically aligning the charger 20 to its optical position without the patient having to find the alignment position by other means. The magnets also hold the charger in its optimal charging positions, so removes the requirement for the patient to be responsible for holding the charger in position for the duration of the charging process. The magnetic alignment of the charger with the IMD should therefore substantially reduce charging time due to increased charging efficiency.

When implanted, the IMD is in tissue 72, usually no more than a few centimeters beneath the skin 70. During recharging, the charger 20 is placed on the surface of the skin 70 in the vicinity of the IMD 1. The patient or a caregiver then slides the charger 20 over the over the area of the IMD 1. When the charger 20 is in a position and is able to couple enough power from its transmit coils 23 into the IMD's receive coils 13, the IMD 1 turns on the electromagnet 7. At this point, the attraction of the magnets 7, 21 causes the charger 20 to provide tactile feedback for optimal positioning. The charger 20 may be made light enough that the magnetic field snaps it into optimal position for recharging, i.e., aligned with the distance '$\delta$' equal to zero and then holds it there for the duration of the charging process.

The charger magnet 21 may be a permanent magnet or an electromagnetic. A permanent magnet is preferred. The IMD magnet 7 may be a permanent magnet or an electromagnet but is preferably an electromagnet which is energized selectively only for charging. One advantage of using a selectively activated electromagnet for the IMD magnet 7 is to make the IMD 1 safe for magnetic resonance imaging (MRI) where a high magnetic field is generated. MRI compliance is an important requirement for IMDs. The IMD electromagnet 7 may be energized, i.e., powered, by the charger 20 through the IMD charging circuity in that a portion of the inductive power received wirelessly from the external charger 20 via the receive coils 13 is used for powering the IMD electromagnet 7. This slightly decreases the energy efficiency of the charging system for charging the IMD rechargeable battery 17. However, this slight decrease is more than offset by the improved efficiency provided by the magnetic locking of the charger to the IMD which provides the alignment between the transmit and receive coils 23 and 13 of the charger 20 and implanted IMD 1 respectively.

The IMD electromagnet 7 may also be powered up by the IMD 1 using its own battery 17. This is useful during initial alignment. If the IMD receives a signal that an alignment procedure is being carried out, e.g., via wireless communication from the charger 20, the IMD electromagnet 7 is powered temporarily by the IMD battery 17 up to a maximum period of time which is limited to avoid draining the IMD battery 17 (e.g. 30 seconds) until such time as it is sensed that the IMD 1 is aligned with the charger 20 at which point the controller process switches to power the IMD electromagnet 7 from the inductive recharging link established between the charger and IMD.

The advantage of temporarily powering the IMD electromagnetic for alignment is to provide a magnetic field to aid the alignment before the charger is in position and able to power the IMD electromagnet. With this approach, it is important that there is an appropriate timeout, so that the IMD battery 17 is not inadvertently depleted should the patient repeatedly select this 'alignment' mode without actually recharging the IMD. The alignment mode to temporarily power up the IMD electromagnet 7 from the IMD battery 17 can be selected via a mobile phone application ('app') installed on the patient remote 32, a connected patient monitor, by a button on the charger 20 or by a button on an external charging unit carrying a battery pack as described below. In all of these the cases, a message is relayed to the IMD 1 through telemetry, and the IMD 1 then powers the electromagnetic 7 temporarily from its battery 17.

In principle, an external magnetic field, such as from an MRI scanner, could provide sufficient power to energize the IMD electromagnetic 7. To mitigate against this risk of this occurring, the IMD controller is configured to energize the IMD electromagnetic 7 conditional on some handshaking protocol taking place with the charger 20 so that the IMD 'knows' that the magnetic field is coming from an authorized charger and is not from an arbitrary external magnetic field source, such as an MRI scanner. For example, the charger 20 may send a unique message, which may be encrypted, to the IMD 1. The communication protocol may be a cryptographic challenge response exchange, such as with public-private key pairs. The communication may be direct near-field communication between the IMD 1 and charger 20 or communication via WPAN links between the IMD 1 and charger 20. Another option is for the charger 20 to modulate the charging field itself so that the IMD 1 can recognize the magnetic field as originating from an authorized charger 20. Only when the correct signal is received by the IMD 1 will the IMD controller allow power from its charging circuitry to flow to the IMD electromagnet 7. Since the MRI's gradient fields can also couple directly into the electromagnet 7, it is important that the switch that disconnects the electromagnet 7 is specified to be able to handle the maximum induced voltages and currents that can be expected to be generated by an MRI scanner.

The IMD electromagnet 7 may be implemented as a solenoid, wrapped around a small diameter core of ferrite or other high permeability material. Although the high permeability core has magnetic properties and will result in a force and/or torque on the IMD 1 in the presence of an MRI scanner, the core is very small, and therefore the force and/or torque can be kept below acceptable limits. The solenoid is arranged in the IMD 1 such that its length orientation will lie perpendicular to the plane of the skin surface when the IMD 1 is implanted. In a flat format IMD, the solenoid length orientation will be perpendicular to the two flat sides of the IMD casing. This orientation will put one magnetic pole at the proximal surface of the IMD 1 (the surface closest to the skin), and the opposite pole at the distal surface of the IMD 1 (the surface furthest from the skin). To maximize the magnetic field resulting from a given applied current, the solenoid should be as long as possible for the given IMD geometry, it should also have as high a number of turns as practical. To maximize the magnetic field, it should have a core of as large a diameter as is practical, and the core should have as high a permeability as is practical.

The magnetic field from a solenoid electromagnet some distance away along the center axis can be approximated with the formula:

$$B = \frac{\mu I R^2}{2} \sum_{n=1}^{N} \frac{1}{z_n{}^3}$$

where μ is the permeability of the core, I is the current, R is the radius of the solenoid, N is the number of turns, and Zn is the distance of each turn from the point at which the magnetic field is being measured.

One specific example specification for the IMD electromagnet is as follows. The solenoid is 8 mm long, has 400 turns of wire, has a 3 mm diameter core with a relative permeability of 1500. At an electromagnet depth of 6 mm from the skin surface, the magnetic field at the skin with a current of 5 mA will be 24 mT..

The magnets are preferably not only powerful enough to provide tactile feedback to the patient on alignment but are also powerful enough to provide a force that helps to "snap" the charger 20 into optimal placement once it is brought near the optimal location. Still further, the magnetic force between the magnets is preferably strong enough to hold the charger in place, i.e., at least providing a magnetic attraction force higher than the gravitational force acting on the charger 20. For the tactile feedback, locking into alignment and holding in place to be effective with modestly specified magnets, it is desirable that the charger 20 is as light as possible. To achieve this, the charger 20 is preferably powered externally, at least to power the IMD charging process, via a power cord. Power may be supplied directly from a mains supply by connecting the power cord to a wall plug. However, preferably power is supplied from a portable unit containing a battery pack, which can be carried in a holster on a belt worn by the patient or in a patient's jacket pocket. The patient thus remains free from wired connection to a wall socket and has freedom of movement during the charging process.

The controller may be implemented such that the IMD electromagnet is de-energized when the IMD battery is fully charged. This will cause the magnetic locking between charger and IMD to cease and the charger will fall away from the skin, providing the patient with a reminder that charging is complete. De-energizing the IMD electromagnet on completion of charging also ensures that the heating of the IMD 1 by eddy currents and the like is also stopped. Unnecessary power drain on the charger battery pack expended on energizing the IMD electromagnet is also avoided (assuming the charger is supplied with power from a battery pack).

One important advantage of the invention is that the charger charging coil 23 can be configured to have a similar size to the IMD charging coil 13, which increases the coupling coefficient and therefore improves charging efficiency. With the magnetic alignment of the invention, the coils will always be near-optimally aligned, so there is no need to design in an assumption that there will most often be a large misalignment between the transmit and receive coils. With the invention, the transmit and receive coils can therefore be designed to be much closer to the same size, e.g., with the transmit coils having a maximum or average lateral dimension that is oversized in relation to the receive coils by a multiple of less than or equal to any one of 1.5, 1.4, 1.3, 1.2 or 1.1. The improvement in charging efficiency achieved by more closely matching the transmit and receive coil maximum or average lateral dimensions, e.g., maximum or average coil diameters in the case of circular coils, is significant given that the coupling coefficient scales with the square of the difference in coil size. The effect may be visualized by considering that more of the field lines from the transmit coils go through the receive coils when the coils are of similar size. Here it is noted that the coils need not be circular but may be square, rectangular, other polygonal shape (in each case optionally with rounded corners), ellipsoid or some other shape.

The charging process is controlled taking account of temperature readings provided by one or more temperature sensors 28, 12 placed at locations on the charger 20 and the IMD 1 where the temperature is expected to be highest during charging, thereby to keep the thermal dose within specified limits. The charger 20 may include a controller 25 loaded with machine readable instructions to control the charging of an IMD 1. The IMD 1 charging process may be controlled by the controller 25 according to the machine-readable instructions dependent on the temperatures measured by the temperature sensors 28, 12 built into the charger and/or IMD 1. The charger 20 may include a wireless transceiver 26 operable to receive IMD temperature data from the IMD 1 being charged, wherein the charging of the implanted IMD 1 is controlled by the controller according to the machine-readable instructions dependent on the IMD temperature data. It is therefore possible to control the charging process taking account of various temperature readings from different locations in the charger 20 and the IMD 1.

The charger 20 can be provided with a temperature sensor 28 in order to measure skin temperature during charging. The IMD 1 is provided with temperature sensors 12 located where it can monitor the temperature of the part of the surface of the IMD 1 is expected to become hottest during charging. In one embodiment, the temperature sensor is not directly monitoring the hottest section of the surface of the IMD 1 (because, for example, it is not practical to place a temperature sensor in that exact location) but instead indirectly monitoring that temperature by monitoring a different location and scaling the temperature appropriately. For example, A temperature sensor 12 can be arranged on the proximal side of the metal casing of the can 4, which is the closest part of the metal casing to the transmit coil 23 so will be heated the most from eddy currents. A further temperature sensor 12 can be arranged on the proximal side of the rechargeable battery, which has a metal jacket that is also prone to eddy current heating during charging. A still further temperature sensor 12 can be arranged adjacent the receive coils 13.

For safety, it is important to limit the heating of the metal outer casing that takes place during wireless charging, since it is in direct contact with the patient's tissue. The industry standard ISO 14708-3:2017 specifies temperature limits. The definition is based on a thermal dose limit expressed in cumulative equivalent minutes at 43°C (referred to as CEM43), this being approximately 6°C above normal body temperature. The CEM43 dose is defined by the following integral.

$$CEM43°C = \int_0^t d\tau R^{43-T(\tau)}$$

$$R = \begin{cases} 0.5 & if\ T \geq 43°C \\ 0.25 & if\ T < 43°C \end{cases}$$

As seen from the above formula, the CEM43 dose increases exponentially when the temperature exceeds 43°C. The choice of this value is made, since prolonged exposure to temperatures above 43°C causes tissue damage. The general aim when wireless charging is to maximize the charging rate of the IMD battery while keeping the thermal dose within the dose limit. To avoid overheating, it is known to provide a temperature sensor in the IMD to measure the temperature of the metal casing so that charging is paused, or at least charging power is reduced, whenever the metal casing becomes too hot, e.g., too close to 43°C, and then restarted, or increased, after the metal casing has cooled sufficiently, e.g., to a temperature having a certain delta below 43°C.

Temperature signals or values obtained by IMD temperature sensors 12 are transmitted to the charger 20, for example via transmission from the IMD with wireless personal area network (WPAN) transceiver 26 to the patient remote controller and from the patient remote controller to the charger, or directly via WPAN or a near-field communication link established between the IMD and the charger. The charger controller 25 therefore takes account of the temperature readings to control charging to ensure: the thermal dose complies with ISO 14708-3:2017 (or some other specified standard or criterion); the skin is not heated excessively, e.g., at no time exceeds 43°C; and no part of the IMD 1 whose temperature is being measured exceeds a certain temperature, e.g., at no time exceeds 43°C. In use, when the IMD 1 is becoming too hot, for example due to charger induced eddy currents on the surface of the IMD, the charger controller 25 can intervene to reduce charging power. Any suitable control algorithm may be used, for example PID (Proportional - Integral - Derivative) control.

The charger 20 may also include a visual indicator 30, such as a display and/or indicator lights, to convey information to the patient, for example on the charging status of the IMD battery 17, the progress of the charging process, and the charger configuration parameters. According to an embodiment, an additional indication is for alignment of the transmit and receive coils, which may be binary (aligned/not aligned) with red and green lights, or more a graduated scale showing how well aligned the coils are. Another useful indication is charging level (e.g. with bars or by displaying a percentage value) and/or a countdown of expected remaining charging time to full charge. Equivalent audible indicators may also be provided, which may for example be useful if the patient is aligning the charger on their back or somewhere else where there is no line of sight to the IMD.

**Reference numerals**

| 1 | implantable medical device (IMD) |
|---|---|
| 2 | header |
| 3 | header outer casing, e.g., ceramic, plastics, epoxy |
| 4 | can |
| 5 | can outer casing, e.g. titanium or titanium alloy |
| 6 | lead attachment ports |
| 7 | magnet, e.g., electromagnet |
| 8 | electronics unit |
| 10 | system-on-a-chip (SoC) |
| 12 | temperature sensor |
| 13 | receive coils |
| 14 | wireless personal area network (WPAN) transceiver |
| 16 | driver circuit, e.g., SCS device with attached leads |
| 17 | rechargeable battery |
| 20 | charger |
| 21 | charger magnet |
| 22 | charger rechargeable battery |
| 23 | charger transmit coils |
| 24 | charger external power connection |
| 25 | charger controller, e.g., microcontroller |
| 26 | charger WPAN transceiver |
| 28 | skin temperature sensor |
| 30 | charger visual indicator, e.g., display |
| 32 | patient remote controller (patient remote) |
| 34 | smartphone wireless personal area network (WPAN) transceiver |
| 35 | smartphone cellular transceiver |
| 36 | smartphone wireless local area network (WLAN) transceiver |
| 40 | router |
| 45 | outer internet connection |
| 50 | internet connection |
| 55 | cellular network base station (cellular tower) |
| 60 | remotely located data center (backend) |
| 65 | portal |
| 70 | patient's skin |
| 72 | patient's tissue |
| $80_1$ to $80_N$ | leads |
| 90.a to 90.h | electrodes |
| 100 | medical device communication system (MDCS) |

**Claims**

1. An implantable medical device, IMD (1) comprising

   an electronics unit (8) for controlling the IMD;
   a rechargeable battery (17) connected to power the electronics unit;
   receive coils (13) connectable by the electronics unit to charge the rechargeable battery and arranged to receive an electromagnetic field to provide electrical energy for wirelessly charging the rechargeable battery (17); and
   a magnet (7) configured to provide a magnetic field to couple with a corresponding magnet of an external charger when placed adjacent the IMD (1).

2. The IMD (1) of claim 1, wherein the magnet is an electromagnet.

3. The IMD (1) of claim 2, wherein the electronics unit is configured to energize the electromagnet with electrical power received by the receive coils inductively from an external charger.

4. The IMD (1) of claim 2 or 3, wherein the electronics unit is configured to selectively connect the rechargeable battery to the electromagnet to energize the electromagnet.

5. The IMD (1) of claim 4, wherein the electronics unit is configured to sense when electrical power is being received by the receive coils inductively from an external charger and responsive thereto to disconnect the rechargeable battery from the receive coils.

6. The IMD (1) of any one of claims 2 to 5, wherein the electronics unit includes a switch to selectively allow or prevent the electromagnet from being energized.

7. The IMD (1) of claim 6, wherein the electronics unit is configured to sense the charge state of the rechargeable battery and in response thereto if it is sensed that the rechargeable battery is fully charged to actuate the switch to prevent the electromagnet from being energized.

8. The IMD (1) of claim 6, wherein the electronics unit is configured to actuate the switch to allow the electromagnet to be energized conditional on receipt of a predefined communication signal from an external charger.

9. The IMD (1) of any one of the preceding claims, wherein the magnet is arranged centrally in the receive coils.

10. A charger (20) for wirelessly charging an implantable medical device, IMD (1) that is implanted in a patient, the charger comprising:

> transmit coils (23) arranged to generate an electromagnetic field for wirelessly charging an implanted IMD (1); and
> a magnet (21) configured to provide a magnetic field to couple with a corresponding magnet in an IMD (1) to be charged.

11. The charger of claim 10, wherein the magnet is a permanent magnet.

12. The charger of claim 10 or 11, wherein the magnet is arranged centrally in the transmit coils.

13. A medical device system comprising:

> an implantable medical device, IMD (1) according to any one of claims 1 to 9; and
> a charger according to any one of claims 10 to 12.

14. The medical device system of claim 13, wherein the respective magnets of the charger and IMD (1) are paired to provide a magnetic attraction force sufficient to provide a patient with tactile feedback of alignment when the IMD (1) is implanted subcutaneously in a patient and the charger is arranged over the implanted IMD (1).

15. The medical device system of claim 13 or 14, wherein the respective magnets of the charger and IMD (1) are paired to provide a magnetic attraction force in relation to the weight of the charger that is sufficient to hold the charger in place against gravity when the IMD (1) is implanted subcutaneously in a patient at a specified depth and the charger is aligned over the implanted IMD (1).

16. The medical device system of claim 13, 14 or 15, wherein the transmit and receive coils have respective lateral dimensions, and wherein the transmit coil lateral dimension is less than or equal to a multiple of the receive coil lateral dimension, the multiple being selected from the group: 1.5, 1.4, 1.3, 1.2 and 1.1.

FIG. 1

EP 4 696 368 A1

**FIG. 2A**

FIG. 2B

FIG. 2C

EP 4 696 368 A1

EP 4 696 368 A1

Europäisches
Patentamt
European
Patent Office
Office européen
des brevets

# EUROPEAN SEARCH REPORT

Application Number

EP 24 19 4441

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/126574 A1 (PRECISIS GMBH [DE]) 20 June 2024 (2024-06-20) * abstract; figures 1-3, 5 * * page 5, line 1 - page 30, line 25 * ----- | 1-16 | INV. A61N1/37 A61N1/378 |
| A | US 2017/095667 A1 (YAKOVLEV ANATOLY [US] ET AL) 6 April 2017 (2017-04-06) * the whole document * ----- | 1-16 | |
| A | US 2016/331956 A1 (YAKOVLEV ANATOLY [US] ET AL) 17 November 2016 (2016-11-17) * the whole document * ----- | 1-16 | |

TECHNICAL FIELDS
SEARCHED (IPC)

A61N

The present search report has been drawn up for all claims

| Place of search | Date of completion of the search | Examiner |
|---|---|---|
| Munich | 28 January 2025 | Wetzig, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.............................................................................................
& : member of the same patent family, corresponding document

EPO FORM 1503 03.82 (P04C01)

13

## ANNEX TO THE EUROPEAN SEARCH REPORT
## ON EUROPEAN PATENT APPLICATION NO.

EP 24 19 4441

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2025

| Patent document cited in search report | Publication date | Patent family member(s) | Publication date |
|---|---|---|---|
| WO 2024126574 A1 | 20-06-2024 | LU 503204 B1<br>WO 2024126574 A1 | 17-06-2024<br>20-06-2024 |
| US 2017095667 A1 | 06-04-2017 | NONE | |
| US 2016331956 A1 | 17-11-2016 | EP 3010581 A1<br>US 2016331956 A1<br>US 2022072300 A1<br>WO 2014205129 A1 | 27-04-2016<br>17-11-2016<br>10-03-2022<br>24-12-2014 |

EPO FORM P0459

For more details about this annex : see Official Journal of the European Patent Office, No. 12/82